# EUROPEAN PATENT APPLICATION

(11) **EP 3 549 593 A1**
(43) Date of publication of application: **09.10.2019**
(21) Application number: 17875934.6
(22) Date of filing: 19.06.2017
(51) Int. Cl.: A61K 35/745, A23L 33/00, A23L 33/135, A61K 31/7048, A61K 35/74, A61P 3/06, A61P 5/30, A61P 9/00, A61P 9/10, A61P 19/10, A61P 25/28, A61P 35/00, C12N 1/20, C12P 7/22

(54) **AGLYCONE PRODUCTION PROMOTER**

(30) Priority: 29.11.2016 JP 2016231320
(71) Applicant: Morinaga Milk Industry Co., Ltd., Minato-ku Tokyo 108-8384 (JP)
(72) Inventor: YAO, Ruiqing, Zama-shi Kanagawa 252-8583 (JP); SHIMIZU, Kanetada, Zama-shi Kanagawa 252-8583 (JP); ODAMAKI, Toshitaka, Zama-shi Kanagawa 252-8583 (JP)
(74) Representative: Hoffmann Eitle
(86) International application number: PCT/JP2017/022538
(87) International publication number: WO 2018/100776

(57) **Abstract**

A technique is provided for degrading a resveratrol glycoside, or degrading a resveratrol glycoside and an isoflavone glycoside, to promote the production of an aglycone(s), thereby enhancing the absorption thereof into a living body. A bacterium belonging to the genus *Bifidobacterium* is used as an active ingredient of an aglycone production promoter which is used for promoting the production of an aglycone(s) from a glycoside(s). The aglycone production promoter is incorporated into a pharmaceutical composition for preventing and/or treating a disease for which an aglycone(s) is/are effective, or into a food or beverage composition for producing an aglycone(s).

## Description

### TECHNICAL FIELD

An aglycone production promoter, a food or beverage composition for producing an aglycone(s), and a method of producing an aglycone(s) are provided. More particularly, an aglycone production promoter for promoting the production of resveratrol and/or an isoflavone aglycone, a food or beverage composition for producing resveratrol and/or an isoflavone aglycone, and a method of producing resveratrol and/or an isoflavone aglycone is provided.

### BACKGROUND ART

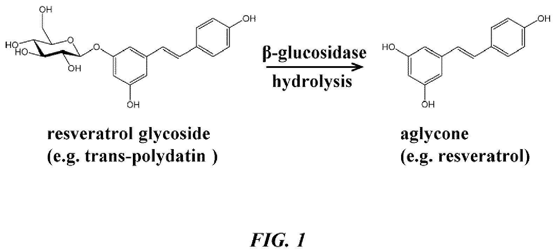

Resveratrol and isoflavones are types of polyphenols, and are known to be contained, for example, in grape skins and soybeans, respectively. Most of resveratrol and isoflavones are present in natural states or in food in the form of glycosides, each composed of a polyphenol skeleton and a sugar chain such as glucose bound to the skeleton. Such a glycoside is not easily absorbed into a living body, as it is; however, when the sugar chain is cleaved due to hydrolysis by an enzyme (β-glucosidase) which is derived from intestinal bacteria or present on the small intestinal microvillous membrane, and the glycoside is converted into an aglycone, as shown in the drawing, the aglycone can be efficiently absorbed into a living body. Therefore, it is desirable that resveratrol or an isoflavone be ingested in the form of an aglycone, rather than in the form of a glycoside, because the pharmacological action thereof can be improved.

For example, Non-patent Document 1 discloses that, after the oral ingestion of daidzein and genistein, which are isoflavone aglycones, the maximum values of the plasma concentrations of the aglycones were five times or greater than those after the ingestion of daidzin and genistin, which are glycosides of daidzein and genistein, respectively. Further, Non-patent Document 1 discloses that the isoflavone aglycones exhibit an excellent effect in the treatment of chronic diseases, such as coronary artery diseases, since isoflavone aglycones are more easily absorbed into a living body as compared to isoflavone glycosides.

β-glucosidase, which serve as a degrading enzyme when an isoflavone glycoside or a resveratrol glycoside is hydrolyzed *in vivo,* is primarily produced by intestinal bacteria. Therefore, the efficiency that the isoflavone glycoside or the resveratrol glycoside which has been taken into a living body is hydrolyzed and absorbed into the body as an aglycone depends on the glycoside-degrading ability (β-glucosidase-producing ability) of the intestinal bacterial flora of each individual. However, since the composition of the intestinal bacterial flora varies between individuals, each individual has a varying degree of glycoside-degrading ability. Accordingly, the absorption efficiency of a glycoside which has been taken into the body varies between individuals. In view of the above, a technique is needed which allows for efficiently degrading a glycoside, regardless of the composition of the intestinal bacterial flora specific to each individual.

So far, various species of bacteria having a glycoside-degrading ability have been reported. For example, Non-patent Document 2 discloses the evaluation results of the β-glucosidase activity, isoflavone glycoside-degrading ability, and metabolic capacity and the like, of total of 92 species of lactic acid bacteria and *Lactobacillus bifidus.*

Patent Document 1 discloses *Lactobacillus casei* strain Hasegawa (FERM P-19484) as a lactic acid bacterium having an ability to hydrolyze a glycoside, and the fact that the lactic acid bacterium exhibited an ability to hydrolyze ginsenoside, which is one type of glycoside.

Furthermore, Patent Document 2 discloses a composition for degrading a glycoside contained in an herbal medicine as an active ingredient, which composition contains: naringinase as a hydrolase; and one or more kinds of cells or cell extracts of bacteria selected from the group consisting of lactic acid bacteria, butyric acid bacteria (*Clostridium butyricum*) and natto bacteria (*Bacillus subtilis var. natto*)*.*

### RELATED ART DOCUMENTS

### PATENT DOCUMENTS

Patent Document 1: JP 2005-160373 A
Patent Document 2: JP 2012-1510 A

### NON-PATENT DOCUMENTS

Non-patent Document 1: Izumi, T. et al., J. Nutr., Vol. 130, No. 7, pp. 1695-1699, (2000)
Non-patent Document 2: Gaya, P. et al., Int. J. Food Sci. Nutr., Vol. 67, No. 2, pp. 117-124, (2016)

### SUMMARY OF THE INVENTION

### PROBLEMS TO BE SOLVED BY THE INVENTION

Although bacteria having an ability to hydrolyze a certain type of glycoside are known, as described above, there have been no reports on intestinal bacteria having an ability to hydrolyze a resveratrol glycoside, and also, there have been no reports yet on intestinal bacteria having a sufficient activity for degrading an isoflavone glycoside.

A technique for degrading a resveratrol glycoside is described, and more particularly, degrading a resveratrol glycoside and an isoflavone glycoside, to promote the production of an aglycone(s), thereby enhancing the absorption thereof into a living body.

### MEANS FOR SOLVING THE PROBLEMS

A bacterium belonging to the genus *Bifidobacterium* that has a high capacity for degrading an isoflavone glycoside and a resveratrol glycoside is described.

In other words, a first aspect of the present invention provides an aglycone production promoter used for promoting the production of an aglycone(s) from a glycoside(s), wherein the aglycone production promoter contains a bacterium belonging to the genus *Bifidobacterium* as an active ingredient, and wherein the aglycone(s) include(s) at least resveratrol.

It is an aspect of the present invention to provide the promoter as described above, wherein the bacterium belonging to the genus *Bifidobacterium* is *Bifidobacterium breve.*

It is an aspect of the present invention to provide the promoter as described above, wherein the aglycone(s) further include(s) an isoflavone aglycone.

It is an aspect of the present invention to provide the promoter as described above, wherein the *Bifidobacterium breve* is one or more selected from the group consisting of *Bifidobacterium breve* ATCC 15700, *Bifidobacterium breve* BCCM LMG 23729, *Bifidobacterium breve* FERM BP-11175 and *Bifidobacterium breve* NITE BP-02460.

It is an aspect of the present invention to provide a pharmaceutical composition for preventing and/or treating a disease for which an aglycone(s) is/are effective, the composition including the aglycone production promoter.

It is an aspect of the present invention to provide a food or beverage composition for producing an aglycone(s), the composition including the aglycone production promoter and a polyphenol glycoside(s), wherein the glycoside(s) include(s) at least a resveratrol glycoside.

It is an aspect of the present invention to provide the food or beverage composition as described above, wherein it is used for promoting the production of an aglycone(s) from a glycoside(s), and wherein the composition includes a bacterium belonging to the genus *Bifidobacterium* as an active ingredient, and the aglycone(s) include(s) at least resveratrol.

It is an aspect of the present invention to provide the food or beverage composition as described above, wherein the bacterium belonging to the genus *Bifidobacterium* contained in the aglycone production promoter is *Bifidobacterium breve.*

It is an aspect of the present invention to provide the food or beverage composition as described above, wherein the glycoside(s) further include(s) an isoflavone glycoside.

It is an aspect of the present invention to provide the food or beverage composition as described above, wherein the *Bifidobacterium breve* is one or more selected from the group consisting of *Bifidobacterium breve* ATCC 15700, *Bifidobacterium breve* BCCM LMG 23729, *Bifidobacterium breve* FERM BP-11175 and *Bifidobacterium breve* NITE BP-02460.

It is an aspect of the present invention to provide a method of producing an aglycone(s), the method including the steps of: culturing a bacterium belonging to the genus *Bifidobacterium* in the presence of a glycoside(s); and collecting the aglycone(s) produced in a culture obtained by culturing the bacterium; wherein the glycoside(s) include(s) at least a resveratrol glycoside.

It is an aspect of the present invention to provide the method of producing an aglycone(s) as described above, wherein the bacterium belonging to the genus *Bifidobacterium* is *Bifidobacterium breve.*

It is an aspect of the present invention to provide the method of producing an aglycone(s) as described above, wherein the glycoside(s) further include(s) an isoflavone glycoside.

It is an aspect of the present invention to provide the method of producing an aglycone(s) as described above, wherein the *Bifidobacterium breve* is one or more selected from the group consisting of *Bifidobacterium breve* ATCC 15700, *Bifidobacterium breve* BCCM LMG 23729 and *Bifidobacterium breve* FERM BP-11175.

It is an aspect of the present invention to provide *Bifidobacterium breve* NITE BP-02460, which is a novel bacterium belonging to the genus *Bifidobacterium.*

It is an aspect of the present invention to provide the use of a bacterium belonging to the genus *Bifidobacterium* in the production of an aglycone production promoter used for promoting the production of an aglycone(s) from a glycoside(s), wherein the aglycone(s) include(s) at least resveratrol.

It is an aspect of the present invention to provide the use of a bacterium belonging to the genus *Bifidobacterium* for promoting the production of an aglycone(s) from a glycoside(s), wherein the aglycone(s) include(s) at least resveratrol.

It is an aspect of the present invention to provide a method of promoting the production of an aglycone(s) from a glycoside(s), the method including the step of culturing a bacterium belonging to the genus *Bifidobacterium* in the presence of the glycoside(s), wherein the aglycone(s) include(s) at least resveratrol.

It is an aspect of the present invention to provide the use of a bacterium belonging to the genus *Bifidobacterium* in the production of a pharmaceutical composition for preventing and/or treating a disease for which an aglycone(s) is/are effective.

It is an aspect of the present invention to provide the use of a bacterium belonging to the genus *Bifidobacterium* for preventing and/or treating a disease for which an aglycone(s) is/are effective.

It is an aspect of the present invention to provide a method of preventing and/or treating a disease for which an aglycone(s) is/are effective, the method including the step of administering a bacterium belonging to the genus *Bifidobacterium* to an animal.

It is an aspect of the present invention to provide the use of a bacterium belonging to the genus *Bifidobacterium* and a polyphenol glycoside(s), in the production of a food or beverage composition for producing an aglycone(s), wherein the glycoside(s) include(s) at least a resveratrol glycoside.

### EFFECT OF THE INVENTION

The absorption of resveratrol and/or an isoflavone into the body can be enhanced and thereby the pharmacological action thereof as a polyphenol(s) can be enhanced.

Furthermore, the bacterium belonging to the genus *Bifidobacterium* is a bacterium originally present in the intestinal bacterial flora of mammals, and, when in its preferred form, the bacterium has a high safety for a living body, and is capable of maintaining the intestinal bacterial flora in a favorable state.

### BRIEF DESCRIPTION OF THE DRAWING

The drawing is a schematic diagram showing the process by which a glycoside is converted into an aglycone under hydrolysis by β-glucosidase.

### MODE FOR CARRYING OUT THE INVENTION

Next, embodiments will be described. It is to be noted, however, that the description is not limited by the following embodiments, and can be varied freely within the scope of the description. The description of percentage is given in percentage by mass, unless otherwise defined.

### < Aglycone Production Promoter >

The aglycone production promoter is used for promoting the production of an aglycone(s) from a glycoside(s), and contains a bacterium belonging to the genus *Bifidobacterium* (hereinafter, also simply referred to as "*Bifidobacterium* bacterium") as an active ingredient. In general, an aglycone is produced when a glycoside is hydrolyzed. The action of β-glucosidase produced by the bacterium belonging to the genus *Bifidobacterium* cleaves the sugar chain binding site of a glycoside and promotes the production of the corresponding aglycone. The aglycone(s) whose production is promoted by the aglycone production promoter is/are polyphenol aglycone(s), and the polyphenol aglycone(s) include(s) at least resveratrol, and can further include(s) an isoflavone aglycone. Furthermore, the glycoside(s) to be targeted by the aglycone production promoter is/are polyphenol glycoside(s), and polyphenol glycoside(s) include(s) at least a resveratrol glycoside, and can further include(s) an isoflavone glycoside. Still further, the glycoside(s) to be targeted by the aglycone production promoter do(es) not include rutin and/or hesperidin, in general.

Also described herein is the use of a bacterium belonging to the genus *Bifidobacterium* in the production of an aglycone production promoter used for promoting the production of an aglycone(s) from a glycoside(s), wherein the aglycone(s) include(s) at least resveratrol.

Also described herein is the use of a bacterium belonging to the genus *Bifidobacterium* for promoting the production of an aglycone(s) from a glycoside(s), wherein the aglycone(s) include(s) at least resveratrol.

Also described herein is a method of promoting the production of an aglycone(s) from a glycoside(s), the method including the step of culturing a bacterium belonging to the genus *Bifidobacterium* in the presence of the glycoside(s), wherein the aglycone(s) include(s) at least resveratrol.

The glycoside(s), the aglycone(s), and the bacterium belonging to the genus *Bifidobacterium* are the same as those described for the aglycone production promoter.

The aglycone production promoter can also be referred to as a glycoside degradation promoter. In other words, since the aglycone production promoter is characterized by containing a bacterium belonging to the genus *Bifidobacterium* as an active ingredient, and the action of β-glucosidase produced by the bacterium enables to cleave the sugar chain binding site of a glycoside(s), the aglycone production promoter can be used for promoting the degradation of the glycoside(s) so as to convert the glycoside(s) into an aglycone(s).

Also described herein is the use of a bacterium belonging to the genus *Bifidobacterium* in the production of a glycoside degradation promoter used for promoting the degradation of a glycoside(s), wherein the glycoside(s) include(s) at least a resveratrol glycoside.

Also described herein is the use of a bacterium belonging to the genus *Bifidobacterium* for promoting the degradation of a glycoside(s), wherein the glycoside(s) include(s) at least a resveratrol glycoside.

The glycoside(s), the aglycone(s), and the bacterium belonging to the genus *Bifidobacterium* are the same as those described for the aglycone production promoter.

The term "glycoside" is used to generally refer to a compound (aglycone) in which a hemiacetal hydroxyl group in a sugar is substituted by a non-sugar compound, and there are various types of glycosides depending on the type of the non-sugar compounds. The "glycoside" usually refers to a polyphenol glycoside. A glycoside containing resveratrol as a non-sugar compound is referred to as a "resveratrol glycoside". Furthermore, a glycoside containing a compound having an isoflavone skeleton as a non-sugar compound is referred to as an "isoflavone glycoside".

The term "aglycone" refers to a non-sugar compound which is produced when a sugar chain is cleaved from a glycoside. When simply referred to as "resveratrol", the resveratrol is an aglycone, unless otherwise defined. Furthermore, an aglycone having an isoflavone skeleton is referred to as an "isoflavone aglycone".

### (1) Bacterium Belonging to the Genus Bifidobacterium

The bacterium belonging to the genus *Bifidobacterium* (hereinafter, also simply referred to as "*Bifidobacterium* bacterium") which can be used may be a known *Bifidobacterium* bacterium, and can be selected arbitrarily as long as the effects are not impaired. Examples the *Bifidobacterium* bacterium include *Bifidobacterium longum, Bifidobacterium bifidum, Bifidobacterium breve, Bifidobacterium adolescentis, Bifidobacterium infantis, Bifidobacterium angulatum, Bifidobacterium animalis, Bifidobacterium pseudolongum* and *Bifidobacterium thermophilum.*

Among these, *Bifidobacterium breve* is an example. As the *Bifidobacterium breve,* a known strain of *Bifidobacterium breve* can be arbitrarily selected. As the *Bifidobacterium breve* , *Bifidobacterium breve* ATCC 15700, *Bifidobacterium breve* BCCM LMG 23729 or *Bifidobacterium breve* FERM BP-11175 can be suitably chosen. Furthermore, it is also possible to use *Bifidobacterium breve* NITE BP-02460, which is a novel bacterium belonging to the genus *Bifidobacterium.*

One species of the bacterium belonging to the genus *Bifidobacterium* may be used alone, or two or more arbitrary species may be used in combination.

Bacteria to which ATCC accession numbers are assigned are available from the American Type Culture Collection (address: 12301 Parklawn Drive, Rockville, Maryland 20852, United States of America).

BCCM LMG 23729 is available from the Belgian Coordinated Collections of Microorganisms (BCCM) (address: Rue De La Science (Wetenschapsstraat) 8, B-1000 Brussels, Belgium), which is a depository institution in Belgium.

FERM BP-11175 has been internationally deposited to the National Institute of Advanced Industrial Science and Technology, Patent Microorganisms Depositary (which is currently the National Institute of Technology and Evaluation, Patent Microorganisms Depositary; postal: 292-0818, address: #120, 2-5-8, Kazusa Kamatari, Kisarazu-shi, Chiba, Japan) on August 25, 2009, under the provisions of the Budapest Treaty, and under the accession No. FERM BP-11175.

DSM 10140 is available from the Deutsche Sammlung von Mikroorganismen und Zellkulturen GmbH (DSMZ) (address: Mascheroder Weg 1b, D-38124 Braunschweig, Germany), which is a depository institution in Germany.

JCM 5820 is available from the Japan Collection of Microorganisms (JCM), National Institute of Physical and Chemical Research, RIKEN Bioresource Center (postal: 305-0074, address: 3-1-1 Takanodai, Tsukuba-shi, Ibaraki), which is a depository institution in Japan.

The *Bifidobacterium breve* NITE BP-02460 is a novel bacterium belonging to the genus *Bifidobacterium,* which is isolated from the intestinal tract of a human infant as an isolation source. To analyze the genetic characteristics of this bacterium, the base sequence of the 16S rRNA gene of the bacterium was identified in a conventional manner. Furthermore, a homology search of the 16S rRNA gene base sequence was carried out for the respective bacteria belonging to the genus *Bifidobacterium,* by BLAST analysis, using the data base of the National Center for Biotechnology Information (NCBI) in the United States.
As a result, it has been confirmed that *Bifidobacterium breve* NITE BP-02460 has a sequence homology of 99% to *Bifidobacterium breve* DSM 20213, which is a reference strain of *Bifidobacterium breve,* and thus, that *Bifidobacterium breve* NITE BP-02460 is a strain of *Bifidobacterium breve* belonging to the genus *Bifidobacterium.*
NITE BP-02460 has been deposited to the National Institute of Technology and Evaluation, Patent Microorganisms Depositary (postal: 292-0818, address: #122, 2-5-8, Kazusa Kamatari, Kisarazu-shi, Chiba, Japan) on April 24, 2017, under the provisions of the Budapest Treaty, and under the accession No. NITE BP-02460.

The content of the bacterium belonging to the genus *Bifidobacterium* in the aglycone production promoter is not particularly limited, and can be set arbitrarily depending on: the frequency and amount to be used of the aglycone production promoter; the content and amount of meals taken by an ingesting individual; the age of the ingesting individual; and the like. For example, the content of the *Bifidobacterium* bacterium in the aglycone production promoter can be from 1 × 10⁶ to 1 × 10¹² CFU/g or from 1 × 10⁶ to 1 × 10¹² CFU/mL, from 1 × 10⁷ to 1 × 10¹¹ CFU/g or from 1 × 10⁷ to 1 × 10¹¹ CFU/mL, or from 1 × 10⁸ to 1 × 10¹⁰ CFU/g or from 1 × 10⁸ to 1 × 10¹⁰ CFU/mL. The above described unit "CFU" is the abbreviation for "colony forming units" and is a unit indicating colony formation.

The aglycone production promoter is expected to have a high safety for a living body, since it contains, as an active ingredient, a bacterium belonging to the genus *Bifidobacterium* which has long been used in food and drugs, and which is present also in the intestines of animals. Therefore, it is considered that the aglycone production promoter is less likely to cause side-effects and dependency, and thus can be ingested continuously for a long period of time.

Furthermore, since the *Bifidobacterium* bacterium is present in the intestines of animals as a so-called good bacterium, it can be expected that the aglycone production promoter provides an effect of controlling the balance in the intestinal bacterial flora.

### (2) Resveratrol and Resveratrol Glycoside

Resveratrol is an aglycone having a structure represented by the following Formula (1). The resveratrol glycoside is not particularly limited, as long as it is a glycoside which contains resveratrol as a non-sugar compound. For example, the resveratrol glycoside may be a trans-polydatin (trans-piceid) represented by the following Formula (2), or a gnemonoside composed of a resveratrol dimer and a sugar chain bound to the dimer.

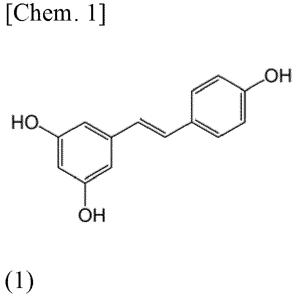

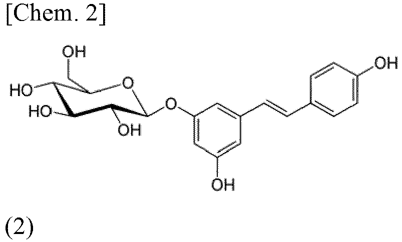

### (3) Isoflavone and Isoflavone Glycoside

In general, flavonoid compounds having a structure represented by the following Formula (3) as a basic skeleton are referred to as isoflavones. Isoflavones encompass isoflavone aglycones and isoflavone glycosides.

An aglycone having the above described basic skeleton, in particular, is referred to as an isoflavone aglycone. Examples of the isoflavone aglycone include daidzein, acetyl daidzein, malonyl daidzein, genistein, acetyl genistein, malonyl genistein, glycitein, acetyl glycitein and malonyl glycitein.

Furthermore, a compound in which a sugar is bound to the above described basic skeleton is referred to as an isoflavone glycoside. The isoflavone glycoside is not particularly limited as long as it is a compound in which a sugar chain is bound to an isoflavone aglycone. Examples of the isoflavone glycoside include a daidzin represented by the following Formula (4), acetyl daidzin, malonyl daidzin, genistin, acetyl genistin, malonyl genistin, glycitin, acetyl glycitin and malonyl glycitin.

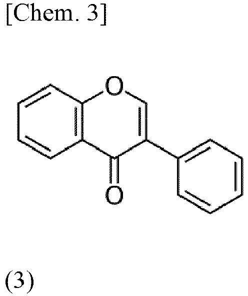

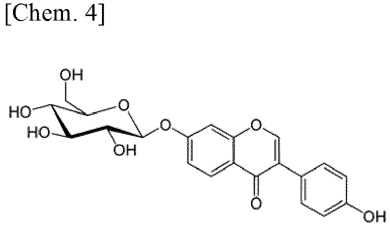

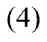

### < Pharmaceutical Composition >

In an embodiment, the aglycone production promoter can be used as a pharmaceutical composition, because the bacterium belonging to the genus *Bifidobacterium* contained therein has a high safety, and provides a favorable effect on the intestinal bacterial flora. The bacterium belonging to the genus *Bifidobacterium* is capable of promoting the production of resveratrol and an isoflavone aglycone which can be absorbed into a living body at a high rate, by degrading a resveratrol glycoside and an isoflavone glycoside.

The pharmaceutical composition can be safely administered even to patients affected by various types of diseases, because the composition contains, as an active ingredient, a bacterium belonging to the genus *Bifidobacterium* which has long been used in food. Furthermore, it is expected that the *Bifidobacterium* bacterium is less likely to cause side-effects, even in cases where the bacterium is continuously administered for a long period of time.

In cases where the aglycone production promoter is used as a pharmaceutical composition, the pharmaceutical composition can be administered either by oral administration or parenteral administration; however, oral administration is a particular example. Examples of the parenteral administration include rectal administration.

In the case of formulating a pharmaceutical composition, it is possible to incorporate any of the components usually used in the formulation of pharmaceuticals, such as a vehicle, a pH adjusting agent, a colorant, a flavoring agent, and the like, in addition to the aglycone production promoter. Furthermore, any known component or component to be found in the future which has an effect of preventing and/or treating a disease for which resveratrol and/or an isoflavone are/is effective, can be used in combination with the bacterium belonging to the genus *Bifidobacterium,* as long as the pharmaceutical composition contains the aglycone production promoter.

The pharmaceutical composition can be formulated in a desired dosage form, as appropriate, depending on the method of administration. For oral administration, for example, the pharmaceutical composition can be formulated in the form of a solid pharmaceutical preparation, such as a powder, granules, a tablet or a capsule; or a liquid preparation such as a solution, a syrup, a suspension or an emulsion. For parenteral administration, the pharmaceutical composition can be formulated in the form of a suppository, an ointment, or the like.

In addition, the formulation can be carried out as appropriate, by a known method and depending on the dosage form. A pharmaceutical carrier may be incorporated as appropriate, in the formulation of the pharmaceutical composition.

In the case of incorporating a pharmaceutical carrier, the content of the bacterium belonging to the genus *Bifidobacterium* is not particularly limited, and it can be selected as appropriate, based on the amount of intake or dose per day, depending on the dosage form. The amount of intake or dose of the *Bifidobacterium* bacterium per 1 kg of body weight per day can be from 1 × 10⁶ to 1 × 10¹² CFU/kg/day, from 1 × 10⁷ to 1 × 10¹¹ CFU/kg/day, or from 1 × 10⁸ to 1 × 10¹⁰ CFU/kg/day.

Furthermore, it is possible to use, as the pharmaceutical carrier, an organic or inorganic carrier, depending on the dosage form. The carrier to be used in the formulation of a solid pharmaceutical preparation may be, for example, a vehicle, a binder, a disintegrating agent, a lubricant, stabilizer or a flavoring agent.

Examples of the vehicle include: sugar derivatives such as lactose, sucrose, glucose, mannitol and sorbitol; starch derivatives such as corn starch, potato starch, α-starch, dextrin and carboxymethyl starch; cellulose derivatives such as crystalline cellulose, hydroxypropyl cellulose, hydroxypropyl methylcellulose, carboxymethyl cellulose and calcium carboxymethyl cellulose; gum arabic; dextran; pullulan; silicate derivatives such as light anhydrous silicic acid, synthetic aluminum silicate and magnesium aluminometasilicate; phosphate derivatives such as calcium phosphate; carbonate derivatives such as calcium carbonate; and sulfate derivatives such as calcium sulfate.

Examples of the binder include, in addition to the above described vehicles: gelatin; polyvinylpyrrolidone; and macrogol.

Examples of the disintegrating agent include, in addition to the above described vehicles: chemically modified starch or cellulose derivatives such as croscarmellose sodium, sodium carboxymethyl starch and crosslinked polyvinylpyrrolidone.

Examples of the lubricant include: talc; stearic acid; metal stearates such as calcium stearate and magnesium stearate; colloidal silica; waxes such as bee gum and spermaceti wax; boric acid; glycols; carboxylic acids such as fumaric acid and adipic acid; sodium salts of carboxylic acids, such as sodium benzoate; sulfates such as sodium sulfate; leucine; lauryl sulfates such as sodium lauryl sulfate and magnesium lauryl sulfate; silicic acids such as anhydrous silicic acid, silicic acid hydrate; and starch derivatives.

Examples of the stabilizer include: paraoxybenzoates such as methylparaben and propylparaben; alcohols such as chlorobutanol, benzyl alcohol and phenyl ethyl alcohol; benzalkonium chloride; acetic anhydride; and sorbic acid.

Examples of the flavoring agent include sweeteners, acidifiers and perfumes.

Examples of the carrier to be used in a liquid preparation for oral administration include: solvents such as water; and flavoring agents.

The aglycone production promoter is useful for degrading a resveratrol glycoside and/or an isoflavone glycoside to promote the production of an aglycone(s). Therefore, by ingesting the aglycone production promoter simultaneously with a resveratrol glycoside and/or an isoflavone glycoside, or by ingesting the aglycone production promoter before or after the ingestion of a resveratrol glycoside and/or an isoflavone glycoside, it is possible to convert the glycoside(s) into an aglycone(s) to enhance the absorption thereof into the body, and to thereby enhance the pharmacological action of resveratrol and/or an isoflavone. Therefore, the aglycone production promoter can be used as a pharmaceutical composition for preventing and/or treating a disease for which an aglycone(s), such as resveratrol and/or an isoflavone aglycone, are/is effective.

In cases where the aglycone production promoter is ingested before or after the ingestion of a resveratrol glycoside and/or an isoflavone glycoside, the aglycone production promoter can be ingested within the time period from 12 hours before the ingestion to five hours after the ingestion of the resveratrol glycoside and/or the isoflavone glycoside, or within the time period from four hours before the ingestion to three hours after the ingestion of the resveratrol glycoside and/or the isoflavone glycoside, or within one hour after the ingestion of the resveratrol glycoside and/or the isoflavone glycoside.

Resveratrol can be used for the prevention and/or treatment of: diseases caused by a decrease in female hormones, such as osteoporosis (J. Feng et al., Acta Biochim. Biophys. Sin., 46 (12), pp. 1024-33, 2014, H. Zhao et al., British Journal of Nutrition, Volume 111, Issue 5, pp. 836-846, 2014), memory decline (H. Zhao et al., Brain Res., Vol. 1467, pp. 67-80, 2012), ischemic cerebrovascular disease (S. I. Jeong et al., Neurobiol Aging., Vol. 44, pp. 74-84, 2016), and brain dysfunction during menopause (H. M. Evans et al., Nutrients, 8(3), 150, 2016); arteriosclerosis (S. B. Vasamsettiet al., Free Radic. Biol. Med., Vol. 96, pp. 392-405, 2016); disorders of lipid metabolism (Tanko Y. et al., Niger. J. Physiol. Sci., 31(1), pp. 71-75, 2016); cardiovascular diseases (Chekalina NI et al., Wiad. Lek., 69 (3 pt 2), pp. 475-479, 2016); and cancers such as skin cancer, breast cancer, prostate cancer, stomach cancer and lung cancer (A. Bishayee et al., Cancer Prev. Res., 2(5), pp. 409-18, 2009).

Accordingly, the pharmaceutical composition can be used for the prevention and/or treatment of: diseases caused by a decrease in female hormones, such as osteoporosis, memory decline, ischemic cerebrovascular disease and brain dysfunction; arteriosclerosis; disorders of lipid metabolism; cardiovascular diseases; cancers such as skin cancer, breast cancer, prostate cancer, stomach cancer and lung cancer; and the like.

On the other hand, isoflavones are known to be effective for the prevention and treatment of: diseases caused by a decrease in female hormones, such as osteoporosis (Y. Tousen et al., British Journal of Nutrition, Volume 116, Issue 2, pp. 247-257, 2016); arteriosclerosis (V. B. Gencel et al., Mini Rev. Med. Chem., Vol.12 (2), pp.149-174, 2012); cancers such as breast cancer, prostate cancer, lung cancer, colon cancer and melanoma (Yoshiko Ishimi, Sachie Ikegami, Journal of Japanese Society of Nutrition and Food Science, Vol. 51, No. 5, pp. 294-298, 1998), and the like.

Accordingly, the pharmaceutical composition can be used for the prevention and/or treatment of: diseases caused by a decrease in female hormones, such as osteoporosis; arteriosclerosis; cancers such as breast cancer, prostate cancer, lung cancer, colon cancer and melanoma; and the like.

Furthermore, the pharmaceutical composition can be used for the prevention and/or treatment of: diseases caused by a decrease in female hormones; arteriosclerosis; cancers such as breast cancer, prostate cancer and lung cancer; and the like, for which both resveratrol and an isoflavone are effective.

Also described herein is the use of a bacterium belonging to the genus *Bifidobacterium* in the production of a pharmaceutical composition for preventing and/or treating a disease for which an aglycone(s) is/are effective.

Also described herein is the use of a bacterium belonging to the genus *Bifidobacterium* for preventing and/or treating a disease for which an aglycone(s) is/are effective.

Also described herein is a method of preventing and/or treating a disease for which an aglycone(s) is/are effective, the method including the step of administering a bacterium belonging to the genus *Bifidobacterium* to an animal. The animal to be administered with the bacterium can be a human.

The aglycone(s), the glycoside(s) corresponding thereto, the bacterium belonging to the genus *Bifidobacterium,* and the disease are the same as those described for the aglycone production promoter and the pharmaceutical composition.

### < Food or Beverage Composition >

The aglycone production promoter can be used as a food or beverage composition for producing an aglycone(s), by processing the aglycone production promoter in the form of a food or beverage, along with a resveratrol glycoside and/or an isoflavone glycoside. In general, the resveratrol glycoside and/or the isoflavone glycoside are subjected to the processing, in the form of raw materials of a food or beverage containing these glycoside(s). In other words, the food or beverage composition can be produced by mixing the bacterium belonging to the genus *Bifidobacterium,* with raw materials of a food or beverage containing a resveratrol glycoside and/or an isoflavone glycoside. Alternatively, the food or beverage composition may be produced by adding the bacterium belonging to the genus *Bifidobacterium,* and a resveratrol glycoside and/or an isoflavone glycoside, to a known food or beverage. Furthermore, it is also possible to produce a new food or beverage composition by mixing the *Bifidobacterium* bacterium and the glycoside(s) into raw materials of a food or beverage.

When such a food or beverage composition is orally ingested, in general, the *Bifidobacterium* bacterium grown in the body (in the intestine) degrades the resveratrol glycoside and/or the isoflavone glycoside, thereby allowing for an efficient aglycone production.

Furthermore, the aglycone production promoter itself may be formed into a food or beverage composition. In other words, a food or beverage containing the *Bifidobacterium* bacterium as an active ingredient can be used, as it is, as a food or beverage composition for promoting the production of an aglycone(s).

The food or beverage composition may be in any form, such as a liquid, a paste, a solid, a powder, or the like. Examples of the form of the composition include: tablet confectioneries, liquid food, feeds (including feeds for companion animals), as well as wheat flour products, instant food, processed agricultural products, processed marine products, processed meat products, milk and dairy products, fats and oils, basic seasonings, complex seasonings and food, frozen food, confectioneries, beverages, and commercially available products other than those described above.

Furthermore, the food or beverage composition can be provided or sold as a food or beverage claiming health benefits, such as, for example, preventing and/or treating a disease for which an isoflavone or resveratrol is effective.

The definition of the act of "claiming" includes all the acts of informing the above described benefits to a user, and any expression which reminds the user of, or enables the user to infer the above described benefits, corresponds to the act of "claiming", regardless of the purpose of claiming, the details being claimed, and the subject or medium for which the benefits are claimed.

Furthermore, the act of "claiming" can be carried out by an expression which enables the user to directly recognize the above described benefits. Specifically, examples of the act of "claiming" include: acts of transferring, delivering, displaying for the purpose of transfer or delivery, and importing a commodity or a commodity package related to a food or beverage on which the above described benefits are described; and acts of displaying or distributing an advertisement, a price list or a transaction document related to the commodity, on which the above described benefits are described, and providing information including these details and the above described benefits via an electromagnetic method (such as internet).

The details being claimed are usually such that they have been approved by the relevant government agency and the like (for example, preferred is a claim which provides information that has been approved under any of various regulations stipulated by the relevant government agency, and which is implemented in a manner in accordance with the approval). Furthermore, the details of such a claim can be provided on advertisement materials to be used at a selling site, such as a package, a container, a catalogue, a pamphlet and a POP, as well as other documents.

The "claim" may be, for example, a claim as: a health food; a functional food; an enteral nutrition food; a food for special dietary uses; a food with health claims; a food for specified health uses; a food with nutrient function claims; a food with function claims; a quasi-drug; or the like. Among these, particularly exemplified is a claim approved by the Consumer Affairs Agency, such as a claim approved by the regulation on food for specified health uses, food with nutrient function claims or food with function claims, or by a regulation similar thereto. Specific examples thereof include: a claim as a food for specified health uses; a claim as a conditional food for specified health uses; a claim claiming an effect on body structure or function; a claim claiming an effect of reducing disease risks, and a functional claim based on scientific grounds. More specifically, typical examples thereof are: a claim as a food for specified health uses (particularly, a claim claiming health benefits) as defined in the Cabinet Office Ordinance (Cabinet Office Ordinance No. 57, issued on August 31, 2009), which is related to the permission of specified health use claims, etc., stipulated in the Health Promotion Act; and a claim similar thereto.

The amounts of the *Bifidobacterium* bacterium as well as the resveratrol glycoside and/or the isoflavone glycoside to be incorporated into the food or beverage composition, at the time of production thereof, are not particularly limited, and can be selected as appropriate depending on the amounts of intake per day. The amount of intake of the *Bifidobacterium* bacterium per 1 kg of body weight per day can be from 1 × 10⁶ to 1 × 10¹² CFU/kg/day from 1 × 10⁷ to 1 × 10¹¹ CFU/kg/day, or from 1 × 10⁸ to 1 × 10¹⁰ CFU/kg/day. Furthermore, the amount of intake or dose of the resveratrol glycoside per day can be from 1 mg/day to 700 mg/day, from 5 mg/day to 200 mg/day, or from 10 mg/day to 20 mg/day. Still further, the amount of intake or dose of the isoflavone glycoside per day can be from 10 mg/day to 120 mg/day, from 15 mg/day to 100 mg/day, or from 20 mg/day to 80 mg/day.

As described above, the aglycone production promoter can be used as a food or beverage composition for a human or an animal, because it has a high safety, and provides a favorable effect on the balance of the intestinal bacterial flora. The aglycone production promoter is capable of enhancing the absorption of an isoflavone or resveratrol into a living body, by promoting the degradation of a resveratrol glycoside and/or an isoflavone glycoside. Therefore, by combining the aglycone production promoter with an isoflavone glycoside or a resveratrol glycoside, the aglycone production promoter can be used as a food or beverage composition for producing an aglycone, which composition is capable of producing an isoflavone or resveratrol. The food or beverage composition can be used for the prevention and/or treatment of a disease for which an isoflavone and/or resveratrol are/is effective.

As described above, resveratrol is known to be effective for the prevention and/or treatment of: diseases caused by a decrease in female hormones, such as osteoporosis, memory decline, ischemic cerebrovascular disease and brain dysfunction; arteriosclerosis; disorders of lipid metabolism; cardiovascular diseases; cancers such as skin cancer, breast cancer, prostate cancer, stomach cancer and lung cancer; and the like.

Therefore, the food or beverage composition can be used for the prevention and/or treatment of: diseases caused by a decrease in female hormones, such as osteoporosis, memory decline, ischemic cerebrovascular disease and brain dysfunction; arteriosclerosis; disorders of lipid metabolism; cardiovascular diseases; cancers such as skin cancer, breast cancer, prostate cancer, stomach cancer and lung cancer; and the like.

Another aspect described herein is the use of a bacterium belonging to the genus *Bifidobacterium* and a polyphenol glycoside(s), in the production of a food or beverage composition for producing an aglycone(s), wherein the glycoside(s) include(s) at least a resveratrol glycoside.

The aglycone(s), the glycoside(s), the bacterium belonging to the genus *Bifidobacterium,* and the applications of the food or beverage composition are the same as those described for the aglycone production promoter, the pharmaceutical composition and the food or beverage composition.

As described above, isoflavones are known to be effective for prevention and/or treatment of diseases caused by a decrease in female hormones, such as osteoporosis; arteriosclerosis; cancers such as breast cancer, prostate cancer, lung cancer, colon cancer and melanoma; and the like.

Therefore, the food or beverage composition can be used for the prevention and/or treatment of: diseases caused by a decrease in female hormones, such as osteoporosis; arteriosclerosis; cancers such as breast cancer, prostate cancer, lung cancer, colon cancer and melanoma; and the like.

Furthermore, the food or beverage composition can be used as a combination of the aglycone production promoter with an isoflavone glycoside and an resveratrol glycoside, and used for the prevention and/or treatment of: diseases caused by a decrease in female hormones; arteriosclerosis; cancers such as breast cancer, prostate cancer and lung cancer; and the like, for which both resveratrol and an isoflavone are effective.

### < Feed >

The aglycone production promoter can be used as a feed. It is possible to prepare a feed having an aglycone-producing effect, by adding the aglycone production promoter, and a resveratrol glycoside and/or an isoflavone glycoside, into a known feed, or alternatively, by mixing the aglycone production promoter, and a resveratrol glycoside and/or an isoflavone glycoside, with raw materials of a feed.

Examples of raw materials of the feed include: grains such as corn, wheat, barley and rye; brans such as bran, wheat bran, rice bran and defatted rice bran; manufacturing cakes such as corn gluten meal and corn germ meal; animal-based feeds such as skim milk, whey, fish powder and bone powder; yeasts such as beer yeast; mineral feeds such as calcium phosphate and calcium carbonate; fats and oils; amino acids; and saccharides. Furthermore, examples of the form of the feed include feeds for companion animals (such as pet food), feeds for farm animals, and feeds for fish.

The amounts of the *Bifidobacterium* bacterium as well as the resveratrol glycoside and/or the isoflavone glycoside to be incorporated into the feed, at the time of production thereof, are not particularly limited, and can be selected as appropriate depending on the amounts of intake per day. The amount of intake of the *Bifidobacterium* bacterium per 1 kg of body weight per day can be from 1 × 10⁶ to 1 × 10¹² CFU/kg/day, from 1 × 10⁷ to 1 × 10¹¹ CFU/kg/day, or from 1 × 10⁸ to 1 × 10¹⁰ CFU/kg/day. Furthermore, the amount of intake or dose of the resveratrol glycoside per day can be from 1 mg/day to 700 mg/day, from 5 mg/day to 200 mg/day, or from 10 mg/day to 20 mg/day. Still further, the amount of intake or dose of the isoflavone glycoside per day can be from 10 mg/day to 120 mg/day, from 15 mg/day to 100 mg/day, or from 20 mg/day to 80 mg/day.

### < Method of Producing Aglycone(s) >

As described above, a method of producing an aglycone(s) is provided.

The above described method includes the steps of: culturing a bacterium belonging to the genus *Bifidobacterium* in the presence of a glycoside(s); and collecting the aglycone(s) produced in a culture obtained by culturing the bacterium. The above described glycoside(s) include(s) at least a resveratrol glycoside.

In the culturing step, the *Bifidobacterium* bacterium produces β-glucosidase, and the glycoside(s) in the culture is/are degraded by the action of the thus produced β-glucosidase, as a result of which resveratrol and/or an isoflavone aglycone can be produced.

Furthermore, the method of producing an aglycone(s) can also be referred to as a method of degrading a glycoside(s).

The *Bifidobacterium* bacterium and a resveratrol glycoside and/or an isoflavone glycoside can be added to a culture medium, and cultured. The glycoside(s) need(s) to be present in the culture medium, at least during a portion of the period during which the *Bifidobacterium* bacterium is cultured. In the culturing step in the method, the glycoside(s) can be added to the culture medium at the start of the culturing and/or during the culturing.

The culture medium is not particularly limited as long as it is capable of culturing a *Bifidobacterium* bacterium, and can be selected as appropriate from known culture media. Specifically, the culture medium may be, for example, an MRS (de Man, Rogosa Sharpe) culture medium.

Furthermore, it is possible to use, as the culture medium, any of various types of milk and dairy products, obtained from: cows, water buffalos, sheep, goats, camels, Indian cattle, Yak cows, horses, donkeys and reindeers; and food such as fermented rice bran for pickling, vegetables, seafood and rice.

The culture can be carried out under known culture conditions which allow for culturing a *Bifidobacterium* bacterium. For example, the culture can be carried out at a temperature of from 25 to 45°C, a temperature of from 30 to 42°C, or from 37 to 42°C.

The culture can be carried out under anaerobic conditions, and it can be carried out under a flow of an anaerobic gas, such as carbon dioxide gas. It is also possible to carry out the culture under microaerophilic conditions, such as, for example, in liquid stationary culture. The culture may be carried out by a method in which the *Bifidobacterium* bacterium alone is cultured first, and the resveratrol glycoside and/or the isoflavone glycoside are/is then added thereto, followed by further culturing.

The culture is carried out for a culturing time of from 1 to 72 hours, and can be adjusted as appropriate while observing the growth rate of the bacterium. However, the culturing time can be from 16 to 48 hours, or from 16 to 24 hours.

In one embodiment, it is possible to separate the aglycone(s) produced as described above from the bacterium, and to collect a fraction containing the aglycone(s). The separation of the aglycone(s) from the bacterium, and the collection of the fraction containing the aglycone(s) from the culture may be carried out simultaneously, or the collection of the fraction containing the aglycone(s) from the culture may be carried out after removing the bacterium from the culture. Alternatively, the aglycone(s) may be directly extracted from the culture by a known extraction method, such as organic solvent extraction.

Examples of the method of removing the bacterium from the culture include filtration using a membrane, and centrifugal separation. The membrane to be used in the filtration may be a flat membrane or a hollow fiber membrane. In cases where the filtration is carried out using a hollow fiber membrane, it is possible to simultaneously carry out the separation of the aglycone(s) from microorganisms, and the collection of the fraction containing the aglycone(s) from the culture.

Furthermore, a known method can be used to carry out the process of collecting the fraction containing the aglycone(s) from the culture from which the bacterium has been removed. Examples of the method include gel filtration, and various types of chromatography methods, such as reverse-phase HPLC. The method to be used can be selected from these methods, as appropriate, depending on the type of aglycone(s) of interest. The chromatography may be a low pressure or high pressure liquid chromatography (HPLC).

The fraction containing the aglycone(s) is not particularly limited as long as the effects of the aglycone(s) are not impaired. The fraction may contain a culture medium component, or may be one which has been partially or completely purified. The purification of the aglycone(s) can be carried out by combining any of the above described methods of collecting the fraction containing the aglycone(s), as appropriate.

The properties of the fraction containing the aglycone(s) are not particularly limited, and the fraction may be a liquid, or a powder obtained by freeze-drying etc.

The aglycone(s) can be incorporated into a composition, such as a pharmaceutical, a quasi-drug, a skin external preparation, a cosmetic, a food or beverage, a food additive or a feed, based on the physiological effect of the aglycone(s). The aglycone(s) to be incorporated into the composition may be either the aglycone(s) which have been isolated and purified, or the fraction containing the aglycone(s).

### EXAMPLES

The present invention will now be described in further detail with reference to Examples. It is noted, however, that the present invention is in no way limited to these Examples.

### [Test Example 1]

A test was carried out to examine whether *Bifidobacterium* bacteria degrade trans-polydatin, which is a resveratrol glycoside, and promote the production of resveratrol.

### (1) Preparation of Culture Broths

Bacterial solutions were prepared, each containing cells of one of the following 12 species of *Bifidobacterium* bacteria which had been cryopreserved in 10% skim milk culture media. A quantity of 100 µL of each of the bacterial solutions was added to 3 mL of an MRS liquid culture medium, and the resulting mixture was anaerobically cultured at 37°C for 16 hours, such that the number of bacterial cells of the *Bifidobacterium* bacterium in each mixture was 1 × 10⁹ CFU/mL. The MRS liquid culture medium was prepared by dissolving 5.5 g of Difco Lactobacilli MRS Broth (manufactured by BD Biosciences) and 50 mg of L-Cysteine Monohydrochloride, Monohydrate (manufactured by Wako Pure Chemical Industries, Ltd.) in pure water to a total volume of 100 mL, and adjusting the pH of the resultant to 6.5 with an aqueous HC1 solution, followed by sterilization at 121°C for 15 minutes.
- *Bifidobacterium longum subsp. Longum* ATCC 15707
- *Bifidobacterium longum subsp. Infantis* ATCC 15697
- *Bifidobacterium breve* ATCC 15700
- *Bifidobacterium breve* BCCM LMG 23729
- *Bifidobacterium breve* FERM BP-11175
- *Bifidobacterium bifidum* ATCC 29521
- *Bifidobacterium adolescentis* ATCC 15703
- *Bifidobacterium angulatum* ATCC 27535
- *Bifidobacterium animalis subsp. lactis* DSM 10140
- *Bifidobacterium pseudolongum subsp. globosum* JCM 5820
- *Bifidobacterium pseudolongum subsp. pseudolongum* ATCC 25526
- *Bifidobacterium thermophilum* ATCC 25525

### (2) Mixed Culture of Trans-polydatin and Bifidobacterium Bacterium

To 100 µL of each of the thus prepared culture broths of the above described 12 species of *Bifidobacterium* bacteria, 0.2 µL of a solution prepared by dissolving trans-polydatin (manufactured by Nagara Science Co., Ltd.) in dimethyl sulfoxide (DMSO) (manufactured by Wako Pure Chemical Industries, Ltd.) to a concentration of 250 mM, was added to prepare a mixed liquid. Thereafter, each mixed liquid was anaerobically cultured at 37°C for 24 hours.

### (3) Quantification of Resveratrol

Each culture broth after the culture was extracted with ethyl acetate, and then the solvent was removed from the extract, followed by drying to obtain a dried product. To the dried product, 25 µL of methanol was added to dissolve the dried product, and 5 µL of the resulting solution was spotted on a silica gel thin-layer chromatography (TLC) plate, and was developed with a developing solvent prepared by mixing toluene and acetone at a ratio of 2:1. After the completion of the development, UV light was irradiated to detect resveratrol. The detection results were analyzed using image analysis software (ImageJ), and resveratrol was quantified by comparison with control and standard samples, and the degradation rate of the glycoside was calculated. The control sample was prepared by mixing 100 µL of the MRS liquid culture medium which does not contain any bacteria, and 0.2 µL of the glycoside solution. Further, a solution prepared by dissolving resveratrol in methanol to a concentration of 2 mM was used as the standard sample.

### (4) Results

The degradation rate of trans-polydatin by each of the 12 species of *Bifidobacterium* bacteria was as shown in Table 1. It was confirmed that each of the *Bifidobacterium* bacteria degraded trans-polydatin to produce resveratrol.

**[Table 1]**

| | Degradation rate of trans-polydatin (%) |
|---|---|
| *Bifidobacterium longum* subsp. *longum* ATCC 15707 | 7.6 |
| *Bifidobacterium longum* subsp. *infantis* ATCC 15697 | 15.8 |
| *Bifidobacterium breve* ATCC 15700 | 77.0 |
| *Bifidobacterium breve* BCCM LMG 23729 | 76.8 |
| *Bifidobacterium breve* FERM BP-11175 | 84.1 |
| *Bifidobacterium bifidum* ATCC 29521 | 6.6 |
| *Bifidobacterium adolescentis* ATCC 15703 | 5.2 |
| *Bifidobacterium angulatum* ATCC 27535 | 18.5 |
| *Bifidobacterium animalis* subsp. *lactis* DSM 10140 | 5.3 |
| *Bifidobacterium pseudolongum* subsp. *globosum* JCM 5820 | 8.5 |
| *Bifidobacterium pseudolongum* subsp. *pseudolongum* ATCC 25526 | 11.8 |
| *Bifidobacterium thermophilum* ATCC 25525 | 12.3 |

### [Test Example 2]

A test was carried out to examine whether the following 11 species of *Bifidobacterium* bacteria degrade daidzin, which is an isoflavone glycoside, and promote the production of daidzein, which is an isoflavone aglycone. In the present test, the same procedure as in Test Example 1 was repeated, except that daidzin (manufactured by Tokyo Chemical Industry Co., Ltd.) was used instead of trans-polydatin used in Test Example 1, and a solution obtained by dissolving daidzein, as an aglycone, in methanol to a concentration of 2 mM was used as the standard sample.
- *Bifidobacterium longum subsp. Infantis* ATCC 15697
- *Bifidobacterium breve* ATCC 15700
- *Bifidobacterium breve* BCCM LMG 23729
- *Bifidobacterium breve* FERM BP-11175
- *Bifidobacterium adolescentis* ATCC 15703
- *Bifidobacterium angulatum* ATCC 27535
- *Bifidobacterium animalis subsp. lactis* DSM 10140
- *Bifidobacterium animalis subsp. animalis* ATCC 25527
- *Bifidobacterium pseudolongum subsp. globosum* JCM 5820
- *Bifidobacterium pseudolongum subsp. pseudolongum* ATCC 25526
- *Bifidobacterium thermophilum* ATCC 25525

As a result, the degradation rate of daidzin by each of the above described 11 species of *Bifidobacterium* bacteria was as shown in Table 2. It was confirmed that each of the *Bifidobacterium* bacteria degraded daidzin to produce daidzein.

**[Table 2]**

| | Degradation rate of daidzin (%) |
|---|---|
| *Bifidobacterium longum* subsp. *infantis* ATCC 15697 | 35.4 |
| *Bifidobacterium breve* ATCC 15700 | 100.0 |
| *Bifidobacterium breve* BCCM LMG 23729 | 93.2 |
| *Bifidobacterium breve* FERM BP-11175 | 100.0 |
| *Bifidobacterium adolescentis* ATCC 15703 | 33.0 |
| *Bifidobacterium angulatum* ATCC 27535 | 60.8 |
| *Bifidobacterium animalis* subsp. *lactis* DSM 10140 | 24.9 |
| *Bifidobacterium animalis* subsp. *animalis* ATCC 25527 | 47.7 |
| *Bifidobacterium pseudolongum* subsp. *globosum* JCM 5820 | 46.2 |
| *Bifidobacterium pseudolongum* subsp. *pseudolongum* ATCC 25526 | 55.4 |
| *Bifidobacterium thermophilum* ATCC 25525 | 21.3 |

### [Test Example 3]

A test was carried out to examine whether *Bifidobacterium breve* NITE BP-02460 degrades trans-polydatin, which is a resveratrol glycoside, and promotes the production of resveratrol, and that the bacterium also degrades daidzin, which is an isoflavone glycoside, and promotes the production of daidzein, which is an isoflavone aglycone. In the present Test Example, the degradation of trans-polydatin was carried out in the same manner as in Test Example 1, and the degradation of daidzin was carried out in the same manner as in Test Example 2, except for the culturing time in the mixed culture. In the present Test Example, the mixed culture of trans-polydatin or daidzin with *Bifidobacterium breve* NITE BP-02460 was carried out at 37°C for one hour or at 37°C for three hours. Resveratrol or daidzein was quantified, after carrying out the mixed culture for each culturing time.

As a result, the degradation rate of trans-polydatin or daidzin by *Bifidobacterium breve* NITE BP-02460 was as shown in Table 3. It was confirmed that *Bifidobacterium breve* NITE BP-02460 degraded trans-polydatin or daidzin to produce resveratrol or daidzein, in either case of carrying out the culture for one hour or for three hours. The results revealed that the effect of promoting aglycone production can be obtained, even if the culture was carried out for a short period of time.

**[Table 3]**

| | Cultured for 1 hour | Cultured for 3 hours |
|---|---|---|
| Degradation rate of trans-polydatin (%) | 19.4 | 29.9 |
| Degradation rate of daidzin (%) | 37.4 | 56.3 |

### [Comparative Test Example 1]

As a Comparative Example, a test was carried out to examine the effect of *Bifidobacterium* bacteria to promote aglycone production, on rutin and hesperidin.

The same procedure as in Test Example 1 was repeated except that rutin (manufactured by Tokyo Chemical Industry Co., Ltd.) or hesperidin (manufactured by Tokyo Chemical Industry Co., Ltd.) was used as a polyphenol glycoside, instead of trans-polydatin used in Test Example 1. Further, as the *Bifidobacterium* bacteria, the following three species were used, which exhibited particularly high degradation rates for trans-polydatin in Test Example 1 and for daidzin in Test Example 2.
- *Bifidobacterium breve* ATCC 15700
- *Bifidobacterium breve* BCCM LMG 23729
- *Bifidobacterium breve* FERM BP-11175

As a result, the presence of an aglycone of rutin or hesperidin was not detected in any of the culture broths containing the respective *Bifidobacterium* bacteria, after the culture. In other words, the results have confirmed that none of the *Bifidobacterium* bacteria has an activity to degrade rutin or hesperidin. This reveals the fact that a *Bifidobacterium* bacterium having an ability to hydrolyze an isoflavone glycoside or a resveratrol glycoside does not necessarily exhibit the same effect of promoting aglycone production, on other polyphenol glycosides.

### [Test Example 4]

A test was carried out to examine the change over time of the degradation of daidzin or trans-polydatin, by the *Bifidobacterium* bacteria which exhibited high degradation rates for trans-polydatin in Test Example 1 and for daidzin in Test Example 2.

### (1) Preparation of Culture Broths

Culture broths were prepared using each of *Bifidobacterium breve* BCCM LMG 23729 and *Bifidobacterium breve* FERM BP-11175, in the same manner as in Test Example 1.

### (2) Mixed Culture of Glycoside and Bifidobacterium bacterium

Each culture broth prepared in (1) and a daidzin solution or a trans-polydatin solution was mixed, and the mixed culture was carried out under the same conditions as in Test Example 1, except that the culture was carried out for four different culturing times: 1, 3, 6 and 24 hours.

### (3) Quantification of Aglycone

Thin-layer chromatography was carried out under the same conditions as in Test Example 1, and the quantification of daidzein or resveratrol was carried out, and the degradation rate of each glycoside was calculated.

### (4) Results

As a result, the change over time of the degradation rate of trans-polydatin was as shown in Table 4. The results revealed that, in each of the cases of using *Bifidobacterium breve* BCCM LMG 23729 and *Bifidobacterium breve* FERM BP-11175, the degradation rate was improved over time, and the degradation of the glycoside proceeded at a relatively short period of time.

**[Table 4]**

| | After 1 hour | After 3 hours | After 6 hours | After 24 hours |
|---|---|---|---|---|
| *Bifidobacterium breve* BCCM LMG 23729 | 18% | 35% | 53% | 65% |
| *Bifidobacterium breve* FERM BP-11175 | 35% | 56% | 71% | 88% |

The change over time of the degradation rate of daidzin was as shown in Table 5. The results revealed that, in each of the cases of using *Bifidobacterium breve* BCCM LMG 23729 and *Bifidobacterium breve* FERM BP-11175, the degradation rate was improved over time, and the degradation of the glycoside proceeded at a relatively short period of time.

**[Table 5]**

| | After 1 hour | After 3 hours | After 6 hours | After 24 hours |
|---|---|---|---|---|
| *Bifidobacterium breve* BCCM LMG 23729 | 24% | 44% | 48% | 87% |
| *Bifidobacterium breve* FERM BP-11175 | 65% | 92% | 100% | - |

### [Test Example 5]

A test was carried out to examine whether the *Bifidobacterium* bacteria actually have an effect of enhancing the absorption of an aglycone into a living body.

Eighteen six-week-old female Wistar rats (obtained from Charles River Laboratories Japan, Inc.) were acclimated for one week, using a feed (F2PLD1) which does not contain any soybean raw material. Thereafter, these rats were divided into three groups each consisting of six rats: a control group in which no *Bifidobacterium* bacterium was administered; Test Group A: in which *Bifidobacterium breve* FERM BP-11175 was administered; and Test group B in which *Bifidobacterium breve* NITE BP-02460 was administered. The rats were then further reared for another week. During this period, 1 mL of a 10% skim milk solution containing 3.0 × 10⁹ CFU/mL of live cells of each corresponding *Bifidobacterium* bacterium was orally administered to each of the rats in the Test Groups A and B, once a day using a sonde. To the rats in the control group, 1 mL of a 10% skim milk solution which does not contain any *Bifidobacterium* bacteria was orally administered once a day, in the same manner.

On the final day of administration (Day 7), and after the administration of each skim milk solution to the rats in each group, an aqueous solution of daidzin (manufactured by Tokyo Chemical Industry Co., Ltd.) prepared to a concentration of 10 mg/mL was orally administered, successively, such that the amount of dose of daidzin to each rat was 50 mg/kg body weight. Before the administration and one hour after the administration, blood was drawn from all the rats.

The thus drawn blood was centrifuged at 4°C and at 10,000 × g for 4 minutes, to obtain plasma samples. To 50 µL of each of the plasma samples, 1 µL of a standard sample containing, as an internal standard, 20 ng of daidzein-d6 (manufactured by Toronto Research Chemicals Inc.) was added. Furthermore, 50 µL of an acetic acid buffer solution (0.2 mol/L, pH: 5.0) containing 100 units of β-glucuronidase (manufactured by Sigma-Aldrich Co. LLC.) was added to the resultant, so that daidzein in the plasma sample, which had been conjugated with glucuronic acid or sulfuric acid when absorbed into a living body, can be separated as an aglycone body. The resulting mixed liquid was then left to stand at 37°C for 15 hours. Thereafter, the mixed liquid was added to 400 µL of methanol, and the resultant was subjected to an ultrasonic treatment and stirred to homogeneity. Subsequently, the resulting liquid was centrifuged at 4°C and at 5,000 × g for 5 minutes, and the supernatant was filtered using a 0.45 µm filtration unit and then collected. The content of daidzein in the supernatant was quantified by LC/MS, to determine the concentration of daidzein in blood. The t-test was carried out to determine a significant difference in the concentration of daidzein in blood one hour after the administration of daidzin, between the Test Groups vs. the control group.

The results were as shown in Table 6. It can be seen from the results that, in the rats in the Test Group A and those in Test Group B to which *Bifidobacterium breve* FERM BP-11175 and *Bifidobacterium breve* NITE BP-02460 were administered, respectively, the concentrations of daidzein in blood one hour after the administration of daidzin are at least 1.3 times that of the rats in the control group, confirming an increase in the blood daidzein concentration. The above results suggest that, by ingesting the glycoside along with the *Bifidobacterium* bacterium, it is possible to enhance the absorption of the corresponding aglycone into a living body.

**[Table 6]**

| | Blood daidzein concentration (ppm) | |
|---|---|---|
| | Before the administration of daidzin | 1 hour after the administration of daidzin |
| Control Group | 0.0069 ± 0.013 | 0.6369 ± 0.1350 |
| Test Group A | 0.0063 ± 0.0003 | 0.8327 ± 0.2090* |
| Test Group B | 0.0065 ± 0.0004 | 0.8811 ± 0.1736** |

| | | |
|---|---|---|
| * 0.05 < P < 0.1: vs. control group (one hour after the administration of daidzin) ** P < 0.05: vs. control group (one hour after the administration of daidzin) | | |

Based on the above results, it can be seen that these *Bifidobacterium* bacteria have an activity to degrade daidzin and trans-polydatin, which are glycosides, and to produce daidzein and resveratrol, which are corresponding aglycones. In particular, *Bifidobacterium breve* FERM BP-11175 degraded 90% or more of daidzin and 50% or more of resveratrol in only three hours, demonstrating its ability to degrade glycosides in a short period of time. Furthermore, it has been found out that, by ingesting the *Bifidobacterium* bacterium along with a glycoside, it is possible to enhance the absorption of a corresponding aglycone into a living body.

## Claims

1. An aglycone production promoter used for promoting the production of an aglycone(s) from a glycoside(s),
wherein the aglycone production promoter comprises a bacterium belonging to the genus *Bifidobacterium* as an active ingredient, and
wherein the aglycone(s) comprise(s) at least resveratrol.

2. The aglycone production promoter according to claim 1, wherein the bacterium belonging to the genus *Bifidobacterium* is *Bifidobacterium breve.*

3. The aglycone production promoter according to claim 1 or 2, wherein the aglycone(s) further comprise(s) an isoflavone aglycone.

4. The aglycone production promoter according to claim 2 or 3, wherein the *Bifidobacterium breve* is one or more selected from the group consisting of *Bifidobacterium breve* ATCC 15700, *Bifidobacterium breve* BCCM LMG 23729, *Bifidobacterium breve* FERM BP-11175 and *Bifidobacterium breve* NITE BP-02460.

5. A pharmaceutical composition for preventing and/or treating a disease for which an aglycone(s) is/are effective, the composition comprising the aglycone production promoter according to any one of claims 1 to 4.

6. A food or beverage composition for producing an aglycone(s), the composition comprising the aglycone production promoter according to claim 1 and a polyphenol glycoside(s), wherein the glycoside(s) comprise(s) at least a resveratrol glycoside.

7. The food or beverage composition for producing an aglycone(s), according to claim 6, wherein the bacterium belonging to the genus *Bifidobacterium* contained in the aglycone production promoter is *Bifidobacterium breve.*

8. The food or beverage composition for producing an aglycone(s), according to claim 6 or 7, wherein the glycoside(s) further comprise(s) an isoflavone glycoside.

9. The food or beverage composition for producing an aglycone(s), according to claim 7 or 8, wherein the *Bifidobacterium breve* is one or more selected from the group consisting of *Bifidobacterium breve* ATCC 15700, *Bifidobacterium breve* BCCM LMG 23729, *Bifidobacterium breve* FERM BP-11175 and *Bifidobacterium breve* NITE BP-02460.

10. A method of producing an aglycone(s), the method comprising the steps of:
culturing a bacterium belonging to the genus *Bifidobacterium* in the presence of a glycoside(s); and
collecting the aglycone(s) produced in a culture obtained by culturing the bacterium;
wherein the glycoside(s) comprise(s) at least a resveratrol glycoside.

11. The method according to claim 10, wherein the bacterium belonging to the genus *Bifidobacterium* is *Bifidobacterium breve.*

12. The method according to claim 10 or 11, wherein the glycoside(s) further comprise(s) an isoflavone glycoside.

13. The method according to claim 11 or 12, wherein the *Bifidobacterium breve* is one or more selected from the group consisting of *Bifidobacterium breve* ATCC 15700, *Bifidobacterium breve* BCCM LMG 23729, *Bifidobacterium breve* FERM BP-11175 and *Bifidobacterium breve* NITE BP-02460.

14. A bacterium belonging to the genus *Bifidobacterium,* which is *Bifidobacterium breve* NITE BP-02460.

15. Use of a bacterium belonging to the genus *Bifidobacterium* in the production of an aglycone production promoter used for promoting the production of an aglycone(s) from a glycoside(s),
wherein the aglycone(s) comprise(s) at least resveratrol.

16. Use of a bacterium belonging to the genus *Bifidobacterium* for promoting the production of an aglycone(s) from a glycoside(s),
wherein the aglycone(s) comprise(s) at least resveratrol.

17. A method of promoting the production of an aglycone(s) from a glycoside(s), the method comprising the step of culturing a bacterium belonging to the genus *Bifidobacterium* in the presence of the glycoside(s),
wherein the aglycone(s) comprise(s) at least resveratrol.

18. Use of a bacterium belonging to the genus *Bifidobacterium* in the production of a pharmaceutical composition for preventing and/or treating a disease for which an aglycone(s) is/are effective.

19. Use of a bacterium belonging to the genus *Bifidobacterium* for preventing and/or treating a disease for which an aglycone(s) is/are effective.

20. A method of preventing and/or treating a disease for which an aglycone(s) is/are effective, the method comprising the step of administering a bacterium belonging to the genus *Bifidobacterium* to an animal.

21. Use of a bacterium belonging to the genus *Bifidobacterium* and a polyphenol glycoside(s), in the production of a food or beverage composition for producing an aglycone(s),
wherein the glycoside(s) comprise(s) at least a resveratrol glycoside.
